# FASCICULE DE BREVET EUROPEEN

(11) **EP 3 927 688 B1**
(45) Date de publication et mention de la délivrance du brevet: **31.01.2024**
(21) Numéro de dépôt: 20710261.7
(22) Date de dépôt: 20.02.2020
(51) Int. Cl.: C07C 407/00, C07C 409/04

(54) **PROCÉDÉ DE PERHYDROLYSE D'ÉPOXYDES ALIPHATIQUES**
VERFAHREN ZUR PERHYDROLYSE ALIPHATISCHER EPOXIDE
PROCESS FOR PERHYDROLYSIS OF ALIPHATIC EPOXIDES

(30) Priorité: 21.02.2019 FR 1901723
(43) Date de publication de la demande: 29.12.2021
(73) Titulaire: Demeta, 35000 Rennes (FR); Université de Rennes, 35042 Rennes (FR)
(72) Inventeur: BORDIER, Corentin, 72120 Saint-Calais (FR); ESCANDE, Vincent, 35000 Rennes (FR); CAIJO, Frédéric, 35235 Thorigne Fouillard (FR); DARCEL, Christophe, 35000 Rennes (FR)
(74) Mandataire: Renard, Emmanuelle
(86) Numéro de dépôt international: PCT/FR2020/050320
(87) Numéro de publication internationale: WO 2020/169932

(56) Documents cités:
- JP-A- 2003 342 255
- YUN LI ET AL: "Facile Ring-Opening of Oxiranes by H2O2 Catalyzed by Phosphomolybdic Acid, incl. Supporting Information", ORGANIC LETTERS, vol. 11, no. 12, 13 mai 2009 (2009-05-13), page 2691, XP055638158, US ISSN: 1523-7060, DOI: 10.1021/ol900811m cité dans la demande
- LIU MIAOMIAO ET AL: "Preparation of Nonanal by Oxidation Cleavage of Methyl Erucate", HUAGONG SHIKAN - CHEMICAL INDUSTRY TIMES, DONGNAN DAXUE, CN, vol. 26, no. 4, 1 January 2012 (2012-01-01), pages 19-22, XP009538796, ISSN: 1002-154X

## Description

### OBJET DE L'INVENTION

La présente invention concerne un procédé de synthèse d'hydroperoxyalcools par perhydrolyse d'époxydes, en utilisant une solution aqueuse de peroxyde d'hydrogène, en présence d'un catalyseur constitué d'acide phosphotungstique.

### ARRIERE-PLAN DE L'INVENTION

Le motif β-hydroperoxy alcool (HPA) est un groupe fonctionnel particulièrement utile en synthèse organique. Il constitue un précurseur permettant la formation d'aldéhydes, de groupes protecteurs de fonctions carbonyles, ou encore de 1,2,4-trioxanes, motif responsable de l'activité pharmacologique de nombreuses molécules à visée antipaludique.¹

La synthèse du motif HPA a été décrite par perhydrolyse d'époxyde, cet époxyde étant soit isolé, soit formé *in situ* par époxydation d'un alcène. La perhydrolyse d'époxyde consiste à faire agir du peroxyde d'hydrogène (soit en solution aqueuse, soit anhydre) en tant que réactif nucléophile sur un époxyde, dont le caractère électrophile est accentué par activation au moyen d'un catalyseur acide.

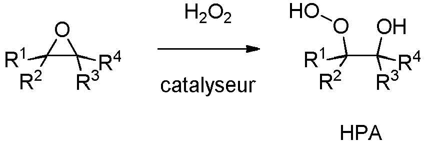

Divers catalyseurs acides, de Bronsted ou de Lewis, ont été décrits pour cette réaction : l'acide tungstique et ses dérivés,^{2,3} les sels de molybdène(VI),^{4,5} l'acide perchlorique,⁶ l'acide trifluoroacétique,' le tétrakis(oxodiperoxotungsto)phosphate,⁸ les sels d'antimoine(III),⁹ ou l'acide phosphomolybdique.¹

Bien que ces méthodes conduisent à la formation du motif HPA, elles souffrent de plusieurs limitations techniques, problématiques dans la perspective d'une application à l'échelle industrielle.

En effet, la production de HPA étant en concurrence avec la formation du diol correspondant, la plupart des méthodes font appel à de l'eau oxygénée à très haute concentration (> 90 % m/V), voire anhydre,^{2,4,6,10} ou même à des extraits éthérés anhydres de peroxyde d'hydrogène,^{1,5,9} afin de défavoriser la formation du diol. Dans ces conditions, des risques importants d'explosion existent et sont rapportés par les auteurs de ces méthodes.^{1,2,9}

Une alternative consiste à utiliser de l'eau oxygénée moins concentrée, mais dans ce cas des taux catalytiques importants sont nécessaires pour favoriser la formation du HPA au détriment du diol.

Des charges catalytiques de l'ordre de 10 % massique sont par exemple classiquement utilisées dans le cas du substrat acide oléique.^{3,8} La demande de brevet JP 2003/342255 décrit ainsi un procédé d'oxydation de l'acide oléique en hydroperoxyalcool correspondant. La réaction est mise en oeuvre en présence de 1 à 20% en poids, par exemple de 10 % en poids, d'un catalyseur tel que H₂WO₄, H₂MoO₄, MoO₃, ou H₃PMo₁₂O₄₀, et de deux équivalents de H₂O₂ à 30-60%, à 35°C et dans un solvant solubilisant à la fois le substrat et le catalyseur, tel que le tert-butanol. Toutefois, des mélanges de l'hydroperoxyalcool et du 1,2-diol correspondant sont formés, et des rendements modérés en hydroperoxyalcool compris entre 4 et 50 % sont obtenus, après des durées de réaction relativement longues, de l'ordre d'au moins cinq heures, voire quinze heures, pour atteindre un taux de conversion d'au moins 75%. La faible activité des catalyseurs utilisés se traduit en outre par une constante catalytique (en anglais, *turnover number*) ou TON (correspondant au ratio de la quantité molaire d'hydroperoxyalcool produit à la quantité molaire de catalyseur) très faible, par exemple de 4 dans le cas de l'acide tungstique.

Ces différents problèmes techniques sont des freins à la préparation industrielle de composés incluant le motif HPA par perhydrolyse d'époxyde.

La publication de Liu Miaomiao et al. (Huagong Shikan - Chemical Industry Times, Dongnan Daxue, vol 26, n° 4, janvier 2012, pp. 19-22) décrit un procédé de synthèse d'hydroperoxyalcools par perhydrolyse d'époxydes à l'aide d'une solution aqueuse de peroxyde d'hydrogène, en présence de tert-butanol et d'un catalyseur constitué d'acide phosphotungstique représentant 3790 ppm molaires par rapport au nombre de moles d'époxyde. Ce procédé ne présente pas des performances catalytiques suffisantes, dans la mesure où le taux de conversion et le rendement voulus sont atteints après un temps de réaction trop long.

Dans ce contexte, il reste nécessaire de développer une méthode de perhydrolyse d'époxydes pouvant satisfaire aux exigences industrielles, à la fois en termes de sécurité et de critères économiques.

Plus précisément, il est souhaitable de proposer une méthode de synthèse conduisant à des rendements élevés en HPA (supérieurs à 60%) en moins de quatre heures, et/ou à des constantes catalytiques élevées (supérieures à 300), et sans utilisation d'eau oxygénée à une concentration dangereuse à manipuler (maximum 70 % m/V).

Les inventeurs ont démontré que ce résultat pouvait être atteint en conduisant la réaction de perhydrolyse sur un substrat époxydé en présence d'un catalyseur particulier, l'acide phosphotungstique H₃PW₁₂O₄₀, utilisé en une quantité maximale qui est cinq fois, voire dix fois, plus faible que la valeur la plus basse préconisée dans le document JP 2003/342255 et en l'absence de solvant organique. Ce procédé peut être mis en oeuvre dans des conditions simples et douces, ne nécessitant pas obligatoirement de chauffage ni le recours à un solvant organique, ce qui constitue des avantages supplémentaires de ce procédé du point de vue économique et environnemental.

### RESUME DE L'INVENTION

L'invention a pour objet un procédé de synthèse d'hydroperoxyalcools de formule (Ia) et/ou (Ib) :

R¹-CR⁴(OOH)-CR³(OH)-R² (Ia)

R²-CR³(OOH)-CR⁴(OH)-R¹ (Ib)

où :
R¹ et R² représentent chacun indépendamment un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe hydroperoxy et/ou hydroxy,
ou bien R¹ et R² forment ensemble un carbocycle constitué de 6 à 12 chaînons et éventuellement substitué,
R³ et R⁴ représentent chacun indépendamment un atome d'hydrogène ou un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe hydroperoxy et/ou hydroxy,
caractérisé en ce qu'il comprend une étape de perhydrolyse de l'époxyde de formule (II) : 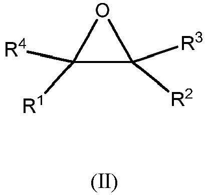 où :
   R¹ et R² représentent chacun indépendamment un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe époxy,
   ou bien R¹ et R² forment ensemble un carbocycle constitué de 6 à 12 chaînons et éventuellement substitué,
   R³ et R⁴ représentent chacun indépendamment un atome d'hydrogène ou un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe époxy,
   par réaction avec une solution aqueuse de peroxyde d'hydrogène, en présence d'un catalyseur constitué d'acide phosphotungstique, ledit catalyseur représentant de 10 à 2000 ppm molaires, par rapport au nombre de moles d'époxyde de formule (II), la réaction de perhydrolyse étant effectuée en l'absence de solvant organique.

### DESCRIPTION DETAILLEE

### Définitions :

Par « alkyle », on entend un groupe hydrocarboné acyclique saturé, linéaire ou ramifié. Des exemples d'alkyle ayant 1 à 6 atomes de carbone (ou « en C₁-C₆ ») sont notamment, un méthyle, un éthyle, un propyle, un isopropyle, un butyle, un tert-butyle, un pentyle, ou un hexyle.

Par « alkylène », on entend un groupe divalent, hydrocarboné acyclique saturé, linéaire ou ramifié. Des exemples d'alkylène ayant 1 à 12 atomes de carbone sont notamment, un méthylène, un éthylène, un propylène, un butylène, un pentylène, un hexylène, un heptylène, un octylène, un nonylène, un décylène, un undécylène, ou un dodécylène.

Par « carbocycle », on entend un groupe hydrocarboné mono- ou polycyclique éventuellement insaturé, aliphatique ou aromatique, de préférence aliphatique. Des exemples de carbocycle ayant 6 à 12 chaînons sont notamment, un cyclohexyle, un cycloheptyle, un cyclooctyle, ou un cyclododécyle.

Par « alcoxy » ou « alkyloxy », on entend un alkyle tel que défini ci-dessus, rattaché au reste de la molécule via une liaison -O- (« -O-alkyle »). Un exemple d'alcoxy est notamment un groupe méthoxy. Par « acyloxy », on entend un groupe de formule -O-C(O)-R" où R" est un groupe hydrocarboné, saturé ou insaturé, linéaire ou ramifié, cyclique ou acyclique, aromatique ou aliphatique. Un exemple de groupe acyloxy est un groupe acétoxy (-O-C(O)-CH₃).

Cette invention porte sur un procédé de synthèse d'hydroperoxyalcools, désignés ci-après par "HPA", suivant une réaction de perhydrolyse d'époxydes.

Les époxydes utilisables dans la présente invention ont pour formule :

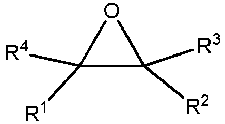

Dans une première forme d'exécution de l'invention, R¹ et R² représentent chacun indépendamment un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe époxyde - CH(O)CH-.

La chaîne alkylène L et les groupes alkyle de R¹, R³ et R⁴ peuvent indépendamment les uns des autres être substitués par au moins un groupe choisi parmi : un groupe hydroxyle, un groupe acyloxy renfermant de 2 à 8 atomes de carbone et un groupe alcoxy renfermant de 1 à 6 atomes de carbone. Des substituants préférés sont les groupes méthoxy, hydroxyle et acétoxy.

On préfère que X désigne un atome d'oxygène. En outre, on préfère que R³ = R⁴ = H.

Lorsque R² désigne un groupement -L-A où A est un atome d'hydrogène, on préfère que L désigne une chaîne alkylène renfermant de 4 à 12, plus préférentiellement de 4 à 8 atomes de carbone et que R¹ représente un groupe alkyle en C₄-C₁₂. Un exemple d'époxyde répondant à cette définition est l'oxyde de tétradéc-7-éne. En variante, L peut représenter une liaison. Un exemple d'un tel époxyde est l'oxyde de octadéc-1-éne.

Dans une mode de réalisation particulier, R¹ ou R² est -L-A où A est un atome d'hydrogène et L est une liaison.

Lorsque R² désigne un groupement -L-A où A désigne un groupe -COXR, on préfère que L désigne une chaîne alkylène renfermant de 5 à 12, plus préférentiellement de 7 à 11 atomes de carbone et que R¹ représente un groupe alkyle en C₆-C₁₀. On préfère que X = O. Le groupement R représente alors avantageusement un atome d'hydrogène ou un groupe méthyle. Des exemples d'époxydes répondant à cette définition sont l'ester méthylique d'acide érucique époxydé (ou 13,14-époxy-docosanoate de méthyle) et l'acide oléique époxydé (ou 9,10-époxy-octadécanoate de méthyle).

En variante, le groupement A peut représenter un groupe -(CH₂)-CH(OCOR⁵)-CHOCOR⁶, où R⁵ et R⁶ désignent chacun indépendamment un groupe alkyle en C₈-C₂₂ éventuellement interrompu par au moins un groupe époxyde -CH(O)CH-. L'époxyde utilisé selon l'invention correspond alors à un triglycéride d'un ou plusieurs acides gras identiques ou différents, de préférence identiques. Un exemple d'un tel composé est le triglycéride d'acide oléique époxydé (ou tri(9,10-époxyoctadécanoate) de glycéryle).

Selon encore une autre possibilité, R² désigne un groupement -L-A où L est une liaison et A désigne un groupe -COXR. X est de préférence un atome d'oxygène. Le groupement R représente alors avantageusement un atome d'hydrogène ou un groupe alkyle en C₁-C₆. Des exemples d'époxydes répondant à cette définition sont l'acide époxymaléique, l'acide époxyfumarique et leurs esters.

Dans une seconde forme d'exécution de l'invention, R¹ et R² forment ensemble un carbocycle constitué de 6 à 12 chaînons qui est éventuellement substitué par au moins un groupe alkyle linéaire ou ramifié en C₁-C₆. Des exemples d'époxydes répondant à cette définition sont donnés ci-dessous :

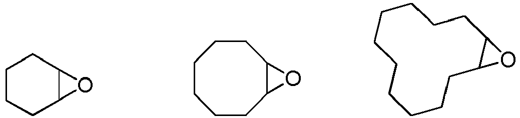

Dans un mode de réalisation particulier de l'invention, l'époxyde de formule (II) est le produit d'époxydation d'un terpène. Les terpènes comprennent notamment les monoterpènes, les sesquiterpènes, les diterpènes, les sesterpènes, les triterpènes, les caroténoïdes ou encore les terpénoïdes. Des exemples de terpènes sont notamment l'α-pinène, le β-pinène, le carène, le limonène, un carotène, l'ocimène, le myrcène, le citronellène, le méthoxycitronellène, le famesène, le squalène, l'astaxanthine, ou encore le 7-méthoxy-3,7-diméthyloct-1-ène, sans que cette liste ne soit limitative.

Dans un mode de réalisation préféré de l'invention, l'époxyde de formule (II) est le produit d'époxydation d'un acide gras mono- ou polyinsaturé ou de son ester, notamment un ester d'alkyle ou un glycéride dudit acide gras. Le composé de formule (II) peut ainsi être choisi parmi les produits d'époxydation de l'acide palmitoléique, de l'acide oléique, de l'acide érucique ou de l'acide nervonique, ou l'un de leurs esters, de préférence le composé de formule (II) est le produit d'époxydation de l'acide oléique ou de l'un de ses esters. L'acide gras ou son glycéride peut lui-même être issu d'une huile végétale. De son côté, l'ester d'alkyle d'acide gras peut être obtenu par transestérification d'au moins une huile végétale.

Comme exemples d'huiles végétales, on peut notamment citer les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

L'époxydation des huiles végétales peut être réalisée de manière classique pour l'homme du métier, de même que leur transestérification éventuelle. On préfère généralement réaliser la transestérification avant l'époxydation.

Dans un autre mode de réalisation, le substrat utilisé dans la réaction de perhydrolyse est le produit d'époxydation d'un alcène. Là encore, l'époxydation peut être effectuée de manière classique pour l'homme du métier, par exemple au moyen d'un peracide carboxylique.

Quel que soit le substrat de type époxyde utilisé, le procédé selon l'invention consiste à transformer l'époxyde en HPA par perhydrolyse, en ajoutant une solution de peroxyde d'hydrogène à l'époxyde, en présence d'un catalyseur particulier qui est constitué d'acide phosphotungstique.

La solution de peroxyde d'hydrogène est concentrée à au moins 30%, voire au moins 45%, et au plus 60%, voire 70%, par exemple à 30%, 45%, 60% ou 70% (m/V). Il a été observé qu'une concentration plus élevée en peroxyde d'hydrogène conduisait à une réaction plus rapide, se traduisant par un taux de conversion d'au moins 90%, de préférence de plus de 95%, voire de plus de 99%, en moins de quatre heures. Le peroxyde d'hydrogène est habituellement mis en oeuvre en une quantité allant de 1 à 1,5 équivalent molaire, de préférence à raison de 1,1 à 1,3 équivalent molaire, par rapport à l'époxyde.

De son côté, le catalyseur est généralement mis en oeuvre en une quantité allant de 0,01 à 2% en poids, par exemple d'au moins 0,1% en poids ou d'au moins 0,2% en poids, notamment d'au moins 0,4% en poids ou d'au moins 0,5% en poids, et par exemple d'au plus 1,5% en poids, notamment d'au plus 1% en poids, par rapport au poids d'époxyde. La quantité de catalyseur mis en oeuvre représente en outre au moins 10 ppm molaires, par exemple au moins 50 ppm molaires voire au moins 100 ppm molaires, notamment au moins 200 ppm molaires, voire au moins 400 ppm molaires ou même au moins 800 ppm molaires et au plus 2000 ppm molaires, avantageusement au plus 1500 ppm molaires, voire au plus 1000 ppm molaires, par rapport à la quantité molaire d'époxyde.

La réaction de perhydrolyse peut être réalisée à une température de 5 à 60°C, de préférence de 20 à 60°C, et plus préférentiellement de 30 à 50°C, pendant une durée allant de 10 minutes à 4 heures, de préférence de 45 minutes à 2h30. Il a été observé que le catalyseur utilisé selon l'invention permettait en effet d'atteindre un taux de conversion de l'époxyde d'au moins 90%, de préférence d'au moins 95%, voire d'au moins 99%, après ce laps de temps (tel que mesuré par RMN ¹H). La durée de la réaction est d'autant plus courte que la température est élevée, à l'intérieur des plages précitées.

Les réactifs précités (époxyde, peroxyde d'hydrogène et catalyseur) peuvent être introduits dans un ordre quelconque dans le réacteur dans lequel la réaction est mise en oeuvre. Par ailleurs, la réaction de perhydrolyse est effectuée en l'absence de solvant organique, c'est-à-dire d'un composé capable de solubiliser l'époxyde et/ou le catalyseur et dont la structure renferme un ou plusieurs atomes de carbone. Des exemples de tels solvants sont notamment les solvants polaires pratiques tels que les alcools. Il a en effet été démontré que ces solvants ralentissaient la réaction de perhydrolyse à température ambiante et diminuaient généralement le rendement en HPA.

Le procédé décrit précédemment permet généralement d'obtenir le HPA recherché avec un rendement d'au moins 60 %, de préférence d'au moins 65%, ou encore d'au moins 70%, généralement d'au moins 80%, voire d'au moins 85% ou même d'au moins 90%. La constante catalytique (TON) de la réaction est par ailleurs toujours supérieure à 300, généralement supérieure à 700 et peut aller jusqu'à 15 000, voire jusqu'à 20 000 ou même 50 000. On obtient généralement un mélange de HPA de formules (Ia) et (Ib).

Ce procédé peut être utilisé dans la coupure oxydante d'huiles végétales époxydées, en particulier pour former des aldéhydes gras et/ou des acides gras. La transformation de l'HPA obtenu selon l'invention en aldéhydes et/ou acides peut être effectuée de manière classique pour l'homme du métier, par catalyse acide ou basique ou par voie radicalaire.

### EXEMPLES

L'invention sera mieux comprise à la lumière des exemples suivants, qui sont donnés à titre purement illustratif et n'ont pas pour but de limiter la portée de l'invention, définie par les revendications annexées.

### Matériel et méthodes

Les réactifs proviennent de fournisseurs commerciaux usuels (Sigma-Aldrich-Merck, Acros, Alfa-Aesar, Fisher) et ont été utilisés sans purification préalable. L'analyse thermogravimétrique révèle que l'acide phosphotungstique hydraté commercial contient 7,87 % (m/m) d'eau. Les quantités de matière indiquées ci-après correspondent donc à l'acide phosphotungstique hydraté commercial. Le 9,10-époxystéarate de méthyle est préparé selon une procédure de la littérature¹¹ et est obtenu avec une pureté de 82 à 99 %.

Analyse RMN : les spectres de Résonance Magnétique Nucléaire (RMN) du proton ont été enregistrés sur un spectromètre RMN AVANCE 400 à 400.1 MHz (Bruker) à 25 °C. Les déplacements chimiques sont exprimés en ppm (partie par million) par rapport au signal du solvant non deutéré résiduel. La multiplicité des signaux est décrite comme suit : singulet (s), doublet (d), triplet (t) et multiplet (m).

Analyse ATG : Les analyses thermogravimétriques ont été effectuées en utilisant l'appareil TGA-DSC-1 Mettler-Toledo sous flux de diazote anhydre avec une vitesse de chauffage de 10 °C/min.

### Exemple 1 : Perhydrolyse du 9,10-époxystéarate de méthyle - Influence de la température

Dans un tube à essai de 6 mL (ø12,25×75×0,80 mm) muni d'un barreau magnétique sont introduits 0,506 mmol de 9,10-époxystéarate de méthyle, 0,48 µmol d'acide phosphotungstique hydraté et 0,617 mmol (35 µL) de solution aqueuse de peroxyde d'hydrogène à 60 % m/V. Le mélange est agité à une température donnée sur une période donnée, indiquées dans le tableau ci-dessous. A la fin de la période d'agitation, une analyse RMN ¹H du brut réactionnel dilué dans 0,5 mL de chloroforme deutéré (CDCl₃) montre que le 9,10-époxystéarate de méthyle est totalement converti en un mélange de 9(10)-hydroperoxy-10(9)-hydroxystéarate de méthyle (HPA) et de 9,10-dihydroxystéarate de méthyle dans les proportions données dans le tableau ci-dessous :

**[Table 1]**

| Temp.^{[a]} (°C) | Temps (min.) | Charge cata. (m%) | Charge cata. (mol ppm) | TON | Conv. (%)^{[a]} | Rend. HPA (%)^{[a]} | Rend. diol (%)^{[a]} | Bilan de masse (%) |
|---|---|---|---|---|---|---|---|---|
| 30 | 60 | 0,968 | 968 | 770 | > 99 | 74 | 12 | 87 |
| 50 | 10 | 0,914 | 913 | 830 | > 99 | 76 | 13 | 89 |

| | | | | | | | | |
|---|---|---|---|---|---|---|---|---|
| ^{[a]} Taux de conversion (Conv.) et rendement (Rend.) déterminés par RMN-¹H du brut réactionnel dilué dans CDCl₃. | | | | | | | | |

Comme le montre cet essai, l'augmentation de la température permet d'obtenir un TON un peu plus élevé en un temps plus court, mais n'a pas d'impact significatif sur le taux de conversion et la sélectivité en HPA, de sorte que la réaction peut être conduite à une température proche de la température ambiante.

### Exemple 2 : Perhydrolyse du 9,10-époxystéarate de méthyle - Influence de la concentration de la solution aqueuse d'eau oxygénée

Dans un tube à essai de 6 mL (ø12,25×75×0,80 mm) muni d'un barreau magnétique sont introduits 0,506 mmol de 9,10-époxystéarate de méthyle, 0,48 µmol d'acide phosphotungstique hydraté et la solution aqueuse de peroxyde d'hydrogène de concentration connue (0,617 mmol). Le mélange est agité à 30 °C sur une période d'une heure. A la fin de l'agitation, une analyse RMN ¹H du brut réactionnel dilué dans 0,5 mL de chloroforme deutéré (CDCl₃) montre que le 9,10-époxystéarate de méthyle est converti en un mélange de 9(10)-hydroperoxy-10(9)-hydroxystéarate de méthyle (HPA) et de 9,10-dihydroxystéarate de méthyle dans les proportions données dans le tableau ci-dessous :

**[Table 2]**

| Concentration H₂O₂ | Charge cata. (m%) | Charge cata. (mol ppm) | TON | Conv. (%)^{[a]} | Rend. HPA (%)^{[a]} | Rend. diol (%)^{[a]} | Bilan de masse (%) |
|---|---|---|---|---|---|---|---|
| (% m/V) | | | | | | | |
| 3 | 0,975 | 975 | 140 | 18 | 13 | 4 | 99 |
| 30 | 0,922 | 922 | 740 | 82 | 68 | 10 | 96 |
| 30^{[b]} | 0,806 | 806 | 910 | > 99 | 73 | 9 | 82 |
| 30^{[c]} | 0,922 | 922 | 890 | > 99 | 82 | 12 | 94 |
| 60 | 0,968 | 968 | 770 | > 99 | 74 | 12 | 87 |
| 70 | 1,040 | 1040 | 770 | > 99 | 79 | 9 | 88 |

| | | | | | | | |
|---|---|---|---|---|---|---|---|
| ^{[a]} Taux de conversion (conv.) et rendement (rend.) déterminés par RMN-¹H du brut réactionnel dilué dans CDCl₃ ^{[b]}2 h 10 de réaction au lieu d'une heure. ^{[c]}16 h de réaction au lieu d'une heure. | | | | | | | |

Il ressort de cet essai que l'utilisation d'une concentration d'eau oxygénée supérieure à 30% (m/V) permet d'atteindre un taux de conversion supérieur à 99% en une heure seulement.

### Exemple 3 : Perhydrolyse du 9,10-époxystéarate de méthyle - Influence de la charge catalytique

Dans un tube à essai de 6 mL (ø12,25x75x0,80 mm) muni d'un barreau magnétique sont introduits 1,050 mmol de 9,10-époxystéarate de méthyle, une quantité connue d'acide phosphotungstique hydraté et 1,280 mmol (72,6 µL) de solution aqueuse de peroxyde d'hydrogène à 60 % m/V. Le mélange est agité à 30 °C sur une période donnée. A la fin de l'agitation, une analyse RMN ¹H du brut réactionnel dilué dans 0,5 mL de chloroforme deutéré (CDCl₃) montre que le 9,10-époxystéarate de méthyle est converti en un mélange de 9(10)-hydroperoxy-10(9)-hydroxystéarate de méthyle (HPA) et de 9,10-dihydroxystéarate de méthyle dans les proportions données dans le tableau ci-dessous :

**[Table 3]**

| Temps réactionnel | Charge cata. (m%) | Charge cata. (mol ppm) | TON | Conv. (%)^{[a]} | Rend. HPA (%)^{[a]} | Rend . diol (%)^{[a]} | Bilan de masse (%) |
|---|---|---|---|---|---|---|---|
| 1 h | 0 | 0 | 0 | 0 | 0 | 0 | 100 |
| 1 h | 0,116 | 116 | 2200 | 29 | 26 | 3 | 100 |
| 29 h 15 | 0,116 | 116 | 5400 | 71 | 63 | 8 | 100 |
| 1 h | 0,242 | 242 | 3300 | 91 | 79 | 10 | 98 |
| 2 h | 0,242 | 242 | 3400 | > 99 | 83 | 11 | 94 |
| 1 h | 0,481 | 481 | 1700 | > 99 | 84 | 11 | 95 |
| 1 h | 0,968 | 968 | 770 | > 99 | 74 | 12 | 87 |
| 1 h | 10,8 | 10800 | 22 | > 99 | 24 | 19 | 43 |

| | | | | | | | |
|---|---|---|---|---|---|---|---|
| ^{[a]} Taux de conversion (Conv.) et rendement (Rend.) déterminés par RMN-¹H du brut réactionnel dilué dans CDCl₃ | | | | | | | |

Il ressort de ce tableau qu'à 30°C, un taux de conversion supérieur à 99% peut être obtenu avec une charge catalytique inférieure à 1% massique et qu'elle peut même être abaissée à 0,2% massique sans allonger substantiellement la durée de la réaction ni affecter le rendement en HPA. En outre, une charge catalytique de l'ordre de 10% massique ne permet pas d'atteindre le rendement voulu en HPA et affecte négativement le TON de la réaction.

### Exemple 4 : Perhydrolyse du 9,10-époxystéarate de méthyle - Etude de la réaction à 50 °C.

Dans un tube à essai de 6 mL (ø12,25x75x0,80 mm) muni d'un barreau magnétique sont introduits du 9,10-époxystéarate de méthyle en quantité variable, 0,110 µmol d'acide phosphotungstique hydraté et une solution aqueuse de peroxyde d'hydrogène à 60 % m/V en quantité variable (1,2 éq. molaire). Le mélange est agité à 50 °C sur une période donnée. A la fin de l'agitation, une analyse RMN ¹H du brut réactionnel dilué dans 0,5 mL de chloroforme deutéré (CDCl₃) montre que le 9,10-époxystéarate de méthyle est converti en un mélange de 9(10)-hydroperoxy-10(9)-hydroxystéarate de méthyle (HPA) et de 9,10-dihydroxystéarate de méthyle dans les proportions données dans le tableau ci-dessous :

**[Table 4]**

| Temps réaction | Charge cata. (m%) | Charge cata. (mol ppm) | TON | Conv. (%)^{[a]} | Rend. HPA (%)^{[a]} | Rend. diol (%)^{[a]} | Bilan de masse (%)^{[a]} |
|---|---|---|---|---|---|---|---|
| 15 min | 0,014 | 14 | 0 | 3 | 0 | 0 | 97 |
| 1 h 10 | 0,014 | 14 | 10700 | 16 | 15 | 1 | 100 |
| 2 h20 | 0,014 | 14 | 11400 | 19 | 16 | 3 | 100 |
| 15 min | 0,028 | 28 | 7200 | 22 | 20 | 2 | 100 |
| 1 h 10 | 0,028 | 28 | 8800 | 27 | 25 | 2 | 100 |
| 2 h 20 | 0,028 | 28 | 6800 | 22 | 19 | 2 | 99 |
| 15 min | 0,055 | 55 | 13800 | 80 | 76 | 4 | 100 |
| 1 h 10 | 0,055 | 55 | 15600 | 90 | 86 | 4 | 100 |
| 15 min | 0,112 | 112 | 7800 | 97 | 88 | 7 | 98 |
| 1 h 10 | 0,112 | 112 | 8300 | > 99 | 93 | 7 | 100 |

| | | | | | | | |
|---|---|---|---|---|---|---|---|
| ^{[a]} Taux de conversion (Conv.) et rendement (Rend.) déterminés par RMN-¹H du brut réactionnel dilué dans CDCl₃ | | | | | | | |

Il ressort de ce tableau qu'à 50°C, un taux de conversion d'au moins 90% peut être obtenu avec une charge catalytique inférieure à 1% massique et qu'elle peut même être abaissée à 0,05% massique sans affecter sensiblement le rendement en HPA.

### Exemple 5 : Perhydrolyse du 9,10-époxystéarate de méthyle - Influence de l'ajout de solvant

Dans un tube à essai de 6 mL (ø12,25x75x0,80 mm) muni d'un barreau magnétique sont introduits 1,23 mmol de 9,10-époxystéarate de méthyle, 0,99 µmol d'acide phosphotungstique hydraté, 1900 µL de *t-*BuOH et une solution aqueuse de peroxyde d'hydrogène à 30% (m/V) (1,25 mmol). Le mélange est agité à une certaine température sur une période donnée. A la fin de l'agitation, une analyse RMN ¹H du brut réactionnel dilué dans 0,5 mL de chloroforme deutéré (CDCl₃) montre que le 9,10-époxystéarate de méthyle est converti en un mélange de 9(10)-hydroperoxy-10(9)-hydroxystéarate de méthyle (HPA) et de 9,10-dihydroxystéarate de méthyle dans les proportions données dans le tableau ci-dessous :

**[Table 5]**

| Temps | Temp (°C) | Charge cata. (m%) | Charge cata. (mol ppm) | TON | Conv. (%)^{[a]} | Rend. HPA (%)^{[a]} | Rend. diol (%)^{[a]} | Bilan de masse (%)^{[a]} |
|---|---|---|---|---|---|---|---|---|
| 2 h 10 | 30 | 0,781 | 781 | 830 | 85 | 65 | 13 | 93 |
| 5 h | 30 | 0,781 | 781 | 1000 | >99 | 78 | 15 | 93 |
| 1 h 05 | 50 | 0,833 | 833 | 890 | 99 | 74 | 18 | 93 |

| | | | | | | | | |
|---|---|---|---|---|---|---|---|---|
| ^{[a]} Taux de conversion (conv.) et rendement (rend.) déterminés par RMN-¹H du brut réactionnel dilué dans CDCl₃ | | | | | | | | |

Comme il ressort de ce tableau, la présence d'un solvant organique dans le milieu réactionnel ne permet pas toujours d'atteindre un taux de conversion maximal de l'époxyde et un rendement acceptable en HPA en environ deux heures maximum. Ce problème peut être surmonté en chauffant légèrement le milieu réactionnel.

En l'absence de solvant, en revanche, un taux de conversion maximal est obtenu en au plus 2h10 avec un rendement acceptable, même à 30°C, comme le montre l'Exemple 2. En outre, il a été observé qu'aux concentrations les plus élevées en peroxyde d'hydrogène, l'absence de solvant permet également d'augmenter nettement le rendement en HPA. En effet, lorsque la réaction de perhydrolyse est conduite dans les conditions de l'Exemple 1, mais en présence de 950 µL de tert-butanol, le rendement en HPA n'est que de 47% (au lieu de 74%) au bout d'une heure à 30°C et 58% (au lieu de 76%) au bout de 10 minutes à 50°C.

### Exemple 6 : Perhydrolyse du 2-(6-méthoxy-6-méthylheptan-2-yl)oxirane

Dans un tube à essai de 6 mL (ø12,25×75×0,80 mm) muni d'un barreau magnétique sont introduits 1,00 mmol de 2-(6-méthoxy-6-méthylheptan-2-yl)oxirane, 0,13 µmol d'acide phosphotungstique hydraté et une solution aqueuse de peroxyde d'hydrogène à 60% (m/V) (1,01 mmol). Le mélange est agité à 1000 tours/min à 50 °C pendant 3 heures. A la fin de l'agitation et à température ambiante, 0,14 mmol d'hexafluorobenzène sont ajoutés au milieu réactionnel, celui-ci servira d'étalon interne pour la quantification des produits réactionnels. Une analyse quantitative RMN ¹³C du brut réactionnel dilué dans 0,5 mL de dichlorométhane deutéré (CD₂Cl₂) montre que le 2-(6-méthoxy-6-méthylheptan-2-yl)oxirane est converti en un mélange composé de 1(2)-hydroperoxy-7-methoxy-3,7-dimethyloctan-2(1)-ol (HPA) et de 7-methoxy-3,7-dimethyloctane-1,2-diol, obtenus avec des rendements de 66% molaire et 34% molaire, respectivement. Un TON de 5 000 est ainsi atteint.

### Exemple 7 : Perhydrolyse du 7-oxabicyclo[4.1.0]heptane

Dans un tube à essai de 19 mL (ø16×150×3,3 mm) muni d'un barreau magnétique sont introduits 5,93 mmol de 7-oxabicyclo[4.1.0]heptane, 0,70 µmol d'acide phosphotungstique hydraté et 0,5 mL de dichlorométhane deutéré. Ce tube est alors placé dans un bain thermostaté à 5 °C puis une lente agitation est appliquée pendant 30 minutes. Passé ce délai, l'agitation est réglée à 1000 tours/min puis une solution aqueuse de peroxyde d'hydrogène à 60% (m/V) (6,00 mmol) est ajoutée lentement au pousse-seringue sur une période de 30 minutes. Enfin, une dernière période d'agitation de 30 minutes à 5 °C permet une conversion totale de l'époxyde. Ensuite, 0,75 mmol de 1,4-dibromobenzène sont ajoutés au milieu réactionnel, celui-ci servira d'étalon interne pour la quantification des produits réactionnels. Une analyse quantitative RMN ¹H du brut réactionnel dilué dans 2 mL de dichlorométhane deutéré (CD₂Cl₂) montre que le 7-oxabicyclo[4.1.0]heptane est converti en un mélange composé de 2-hydroperoxycyclohexan-1-ol (HPA) et de cyclohexane-1,2-diol, obtenus avec des rendements de 34% molaire et 32% molaire, respectivement. Un TON de 2900 est ainsi atteint.

### Exemple 8 : Perhydrolyse d'une huile d'olive époxydée

Dans un tube à essai de 6 mL (ø12,25×75×0,80 mm) muni d'un barreau magnétique sont introduits 332,4 mg d'une huile d'olive époxydée contenant 3,21 mmol/g de fonctions époxydes. Cette composition a été déterminée en quantifiant les groupes fonctionnels alcéniques par RMN ¹H quantitative de l'huile d'olive brute et en s'assurant que la conversion des fonctions alcènes en époxydes a été totale. Sont ensuite introduits 0,13 µmol d'acide phosphotungstique hydraté et une solution aqueuse de peroxyde d'hydrogène à 60% (m/V) (1,06 mmol). Le mélange est agité à 1000 tours/min à 50 °C pendant 4 heures. A la fin de l'agitation et à température ambiante, 1,5 mL de méthanol deutéré et 0,5 mL de chloroforme deutéré sont ajoutés au brut réactionnel puis une analyse quantitative RMN ¹H permet d'observer que l'huile d'olive époxydée s'est complètement transformée en un mélange composé d'hydroperoxyalcools et de diols vicinaux, obtenus avec un rendement de 73 % molaire (en fonctions hydroperoxyalcool) et de 12 % molaire (en fonctions diol vicinal), respectivement. Un TON de 5600 est ainsi atteint.
(1) Li, Y.; Hao, H.-D.; Wu, Y. Facile Ring-Opening of Oxiranes by H2O2 Catalyzed by Phosphomolybdic Acid. Org. Lett. 2009, 11, 2691-2694. https://doi.org/10.1021/ol900811m.
(2) Payne, G. B.; Smith, C. W. Reactions of Hydrogen Peroxide. III. Tungstic Acid Catalyzed Hydroxylation of Cyclohexene in Nonaqueous Media. J. Org. Chem. 1957, 22, 1682-1685. https://doi.org/10.1021/jo01363a042.
(3) Shinichiro, I.; Sunao, N.; Takuji, N.; Hiroko, K. Hydroperoxyhydroxyoctadecanodecanoate Composition and Method for Producing 9-Oxononanoic Acid Dérivative from the Same Composition. JP2003342255 (A), 2003.
(4) Mattucci, A. M.; Perrotti, E.; Santambrogio, A. Epoxidation Reaction with Anhydrous Hydrogen Peroxide. J. Chem. Soc. D 1970, 1198-1199. https://doi.org/10.1039/C29700001198.
(5) Tang, Y.; Dong, Y.; Wang, X.; Sriraghavan, K.; Wood, J. K.; Vennerstrom, J. L. Dispiro-1,2,4-Trioxane Analogues of a Prototype Dispiro-1,2,4-Trioxolane: Mechanistic Comparators for Artemisinin in the Context of Reaction Pathways with Iron(II). J. Org. Chem. 2005, 70, 5103-5110. https://doi.org/10.1021/jo050385+.
(6) Subramanyam, V.; Brizuela, C. L.; Soloway, A. H. Synthesis and Reactions of β-Hydroxyhydroperoxides. J. Chem. Soc. Chem. Commun. 1976, 508-509. https://doi.org/10.1039/C39760000508.
(7) Ogata, Y.; Sawaki, Y.; Shimizu, H. Rates and Scope of the Oxidative Carbon-Carbon Cleavage of Epoxides by Alkaline Hydrogen Peroxide. J. Org. Chem. 1978, 43, 1760-1763. https://doi.org/10.1021/jo00403a029.
(8) Antonelli, E.; D'Aloisio, R.; Gambaro, M.; Fiorani, T.; Venturello, C. Efficient Oxidative Cleavage of Olefins to Carboxylic Acids with Hydrogen Peroxide Catalyzed by Methyltrioctylammonium Tetrakis(Oxodiperoxotungsto)Phosphate(3-) under Two-Phase Conditions. Synthetic Aspects and Investigation of the Reaction Course. J. Org. Chem. 1998, 63, 7190-7206. https://doi.org/10.1021/jo980481t.
(9) Liu, Y.-H.; Zhang, Z.-H.; Li, T.-S. Efficient Conversion of Epoxides into β-Hydroperoxy Alcohols Catalyzed by Antimony Trichloride/SiO2. Synthesis 2008, 3314-3318. https://doi.org/10.1055/s-0028-1083147.
(10) Adam, W.; Rios, A. Perhydrolysis of Epoxides. J. Chem. Soc. D 1971, 822b - 823. https://doi.org/10.1039/C2971000822B.
(11) Deruer, E.; Duguet, N.; Lemaire, M. Thiazolylidene-Catalyzed Cleavage of Methyl Oleate-Derived α-Hydroxy Ketone to the Corresponding Free Aldéhydes. ChemSusChem 2015, 8, 2481-2486. https://doi.org/10.1002/cssc.201500462.

## Revendications

1. Procédé de synthèse d'hydroperoxyalcools de formule (Ia) et/ou (Ib) :
R¹-CR⁴(OOH)-CR³(OH)-R² (Ia)
R²-CR³(OOH)-CR⁴(OH)-R¹ (Ib)
où :
R¹ et R² représentent chacun indépendamment un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe hydroperoxy et/ou hydroxy,
ou bien R¹ et R² forment ensemble un carbocycle constitué de 6 à 12 chaînons et éventuellement substitué,
R³ et R⁴ représentent chacun indépendamment un atome d'hydrogène ou un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe hydroperoxy et/ou hydroxy,
**caractérisé en ce qu'**il comprend une étape de perhydrolyse de l'époxyde de formule (II) : 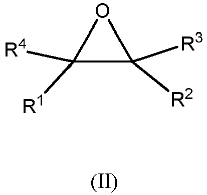 où :
R¹ et R² représentent chacun indépendamment un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe époxy,
ou bien R¹ et R² forment ensemble un carbocycle constitué de 6 à 12 chaînons et éventuellement substitué,
R³ et R⁴ représentent chacun indépendamment un atome d'hydrogène ou un groupement alkyle éventuellement substitué renfermant de 1 à 20 atomes de carbone ou un groupement -L-A où L est une liaison ou une chaîne alkylène linéaire ou ramifiée, renfermant de 1 à 12 atomes de carbone, éventuellement substituée, et A représente un atome d'hydrogène ou un groupe -COXR dans lequel X désigne un atome d'oxygène ou un groupe -NR', et R et R' désignent chacun indépendamment un groupement choisi parmi : un atome d'hydrogène, un groupe alkyle en C₁-C₆ ou un groupe -(CH₂)-CH(OCOR⁵)-CHOCOR⁶, R⁵ et R⁶ désignant chacun indépendamment un groupe alkyle linéaire ou ramifié en C₈-C₂₂ éventuellement substitué et/ou interrompu par au moins un groupe époxy,
par réaction avec une solution aqueuse de peroxyde d'hydrogène, en présence d'un catalyseur constitué d'acide phosphotungstique, ledit catalyseur représentant de 10 à 2000 ppm molaires, par rapport au nombre de moles d'époxyde de formule (II), la réaction de perhydrolyse étant effectuée en l'absence de solvant organique.

2. Procédé selon la revendication 1, **caractérisé en ce que** le catalyseur est mis en oeuvre en une quantité d'au moins 0,01% en poids, de préférence d'au moins 0,1% en poids, notamment d'au moins 0,2% en poids, par exemple d'au moins 0,4% en poids ou d'au moins 0,5% en poids, et par exemple d'au plus 1,5% en poids, notamment d'au plus 1% en poids, par rapport au poids d'époxyde.

3. Procédé selon la revendication 1, **caractérisé en ce que** le catalyseur est mis en oeuvre en une quantité d'au moins 50 ppm molaires, par exemple d'au moins 100 ppm molaires, notamment d'au moins 200 ppm molaires, voire d'au moins 400 ppm molaires ou même d'au moins 800 ppm molaires et d'au plus 2000 ppm molaires, avantageusement d'au plus 1500 ppm molaires, voire d'au plus 1000 ppm molaires, par rapport à la quantité molaire d'époxyde.

4. Procédé selon l'une quelconque des revendications 1 à 3, **caractérisé en ce que** le peroxyde d'hydrogène est mis en oeuvre en une quantité allant de 1 à 1,5 équivalent molaire, de préférence à raison de 1,1 à 1,3 équivalent molaire, par rapport à l'époxyde.

5. Procédé selon l'une quelconque des revendications 1 à 4, **caractérisé en ce que** la réaction de perhydrolyse est mise en oeuvre à une température de 5 à 60 °C, de préférence de 20 à 60 °C, plus préférentiellement de 30 à 50°C, pendant une durée allant de 10 minutes à 4 heures, par exemple de 45 minutes à 2h30.

6. Procédé selon l'une quelconque des revendications 1 à 5, **caractérisé en ce que** l'époxyde de formule (II) est le produit d'époxydation d'un acide gras mono- ou polyinsaturé ou de son ester, notamment un ester d'alkyle ou un glycéride dudit acide gras.

7. Procédé selon la revendication 6, **caractérisé en ce que** l'acide gras est choisi parmi l'acide palmitoléique, l'acide oléique, l'acide érucique et l'acide nervonique, de préférence l'acide gras est l'acide oléique.

8. Procédé selon l'une quelconque des revendications 6 et 7, **caractérisé en ce que** l'acide gras ou son glycéride est issu d'une huile végétale.

9. Procédé selon la revendication 6, **caractérisé en ce que** l'ester d'alkyle d'acide gras est obtenu par transestérification d'au moins une huile végétale.

## Patentansprüche

1. Verfahren zur Synthese von Hydroperoxyalkoholen der Formel (Ia) und/oder (Ib):
R¹-CR⁴(OOH)-CR³(OH)-R² (Ia)
R²-CR³(OOH)-CR⁴(OH)-R¹ (Ib)
obei:
R¹ und R² jeweils unabhängig für eine gegebenenfalls substituierte Alkylgruppe mit 1 bis 20 Kohlenstoffatomen oder eine Gruppe -L-A stehen, wobei L eine Bindung oder eine gegebenenfalls substituierte lineare oder verzweigte Alkylenkette mit 1 bis 12 Kohlenstoffatomen ist und A für ein Wasserstoffatom oder eine Gruppe -COXR steht, in der X ein Sauerstoffatom oder eine Gruppe -NR' bedeutet und R und R' jeweils unabhängig eine Gruppe bedeuten, die aus einem Wasserstoffatom, einer C₁-C₆-Alkylgruppe oder einer Gruppe -(CH₂)-CH(OCOR⁵)-CHOCOR⁶ ausgewählt ist, wobei R⁵ und R⁶ jeweils unabhängig eine lineare oder verzweigte C₈-C₂₂-Alkylgruppe, die gegebenenfalls substituiert und/oder durch mindestens eine Hydroperoxy- und/oder Hydroxygruppe unterbrochen ist, bedeuten,
oder auch R¹ und R² zusammen einen gegebenenfalls substituierten 6- bis 12-gliedrigen Carbocyclus bilden, R³ und R⁴ jeweils unabhängig für ein Wasserstoffatom oder eine gegebenenfalls substituierte Alkylgruppe mit 1 bis 20 Kohlenstoffatomen oder eine Gruppe -L-A stehen, wobei L eine Bindung oder eine gegebenenfalls substituierte lineare oder verzweigte Alkylenkette mit 1 bis 12 Kohlenstoffatomen ist und A für ein Wasserstoffatom oder eine Gruppe -COXR steht, in der X ein Sauerstoffatom oder eine Gruppe -NR' bedeutet und R und R' jeweils unabhängig eine Gruppe bedeuten, die aus einem Wasserstoffatom, einer C₁-C₆-Alkylgruppe oder einer Gruppe -(CH₂)-CH(OCOR⁵)-CHOCOR⁶ ausgewählt ist, wobei R⁵ und R⁶ jeweils unabhängig eine lineare oder verzweigte C₈-C₂₂-Alkylgruppe, die gegebenenfalls substituiert und/oder durch mindestens eine Hydroperoxy- und/oder Hydroxygruppe unterbrochen ist, bedeuten,
**dadurch gekennzeichnet, dass** es einen Schritt der Perhydrolyse des Epoxids der Formel (II): 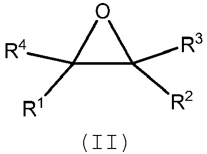
wobei:
R¹ und R² jeweils unabhängig für eine gegebenenfalls substituierte Alkylgruppe mit 1 bis 20 Kohlenstoffatomen oder eine Gruppe -L-A stehen, wobei L eine Bindung oder eine gegebenenfalls substituierte lineare oder verzweigte Alkylenkette mit 1 bis 12 Kohlenstoffatomen ist und A für ein Wasserstoffatom oder eine Gruppe -COXR steht, in der X ein Sauerstoffatom oder eine Gruppe -NR' bedeutet und R und R' jeweils unabhängig eine Gruppe bedeuten, die aus einem Wasserstoffatom, einer C₁-C₆-Alkylgruppe oder einer Gruppe -(CH₂)-CH(OCOR⁵)-CHOCOR⁶ ausgewählt ist, wobei R⁵ und R⁶ jeweils unabhängig eine lineare oder verzweigte C₈-C₂₂-Alkylgruppe, die gegebenenfalls substituiert und/oder durch mindestens eine Epoxidgruppe unterbrochen ist, bedeuten,
oder auch R¹ und R² zusammen einen gegebenenfalls substituierten 6- bis 12-gliedrigen Carbocyclus bilden, R³ und R⁴ jeweils unabhängig für ein Wasserstoffatom oder eine gegebenenfalls substituierte Alkylgruppe mit 1 bis 20 Kohlenstoffatomen oder eine Gruppe -L-A stehen, wobei L eine Bindung oder eine gegebenenfalls substituierte lineare oder verzweigte Alkylenkette mit 1 bis 12 Kohlenstoffatomen ist und A für ein Wasserstoffatom oder eine Gruppe -COXR steht, in der X ein Sauerstoffatom oder eine Gruppe -NR' bedeutet und R und R' jeweils unabhängig eine Gruppe bedeuten, die aus einem Wasserstoffatom, einer C₁-C₆-Alkylgruppe oder einer Gruppe -(CH₂)-CH(OCOR⁵)-CHOCOR⁶ ausgewählt ist, wobei R⁵ und R⁶ jeweils unabhängig eine lineare oder verzweigte C₈-C₂₂-Alkylgruppe, die gegebenenfalls substituiert und/oder durch mindestens eine Epoxidgruppe unterbrochen ist, bedeuten,
durch Reaktion mit einer wässrigen Lösung von Wasserstoffperoxid in Gegenwart eines Katalysators, der aus Wolframatophosphorsäure besteht, umfasst, wobei der Katalysator 10 bis 2000 Mol-ppm, bezogen auf die Zahl der Mole des Epoxids der Formel (II), ausmacht, wobei die Perhydrolysereaktion in Abwesenheit von organischem Lösungsmittel durchgeführt wird.

2. Verfahren nach Anspruch 1, **dadurch gekennzeichnet, dass** der Katalysator in einer Menge von mindestens 0,01 Gew.-%, vorzugsweise mindestens 0,1 Gew.-%, insbesondere mindestens 0,2 Gew.-%, beispielsweise mindestens 0,4 Gew.-% oder mindestens 0,5 Gew.-%, und beispielsweise höchstens 1,5 Gew.-%, insbesondere höchstens 1 Gew.-%, bezogen auf das Gewicht des Epoxids, verwendet wird.

3. Verfahren nach Anspruch 1, **dadurch gekennzeichnet, dass** der Katalysator in einer Menge von mindestens 50 Mol-ppm, beispielsweise mindestens 100 Mol-ppm, insbesondere mindestens 200 Mol-ppm oder sogar mindestens 400 Mol-ppm oder auch mindestens 800 Mol-ppm und höchstens 2000 Mol-ppm, vorteilhafterweise höchstens 1500 Mol-ppm oder sogar höchstens 1000 Mol-ppm, bezogen auf die molare Menge des Epoxids, verwendet wird.

4. Verfahren nach einem der Ansprüche 1 bis 3, **dadurch gekennzeichnet, dass** das Wasserstoffperoxid in einer Menge im Bereich von 1 bis 1,5 Moläquivalenten, vorzugsweise in einer Menge von 1,1 bis 1,3 Moläquivalenten, bezogen auf das Epoxid, verwendet wird.

5. Verfahren nach einem der Ansprüche 1 bis 4, **dadurch gekennzeichnet, dass** die Perhydrolysereaktion bei einer Temperatur von 5 bis 60 °C, vorzugsweise von 20 bis 60 °C, weiter bevorzugt von 30 bis 50 °C, über einen Zeitraum von 10 Minuten bis 4 Stunden, beispielsweise von 45 Minuten bis 2 h 30, durchgeführt wird.

6. Verfahren nach einem der Ansprüche 1 bis 5, **dadurch gekennzeichnet, dass** es sich bei dem Epoxid der Formel (II) um das Produkt der Epoxidation einer ein- oder mehrfach ungesättigten Fettsäure oder eines Esters davon, insbesondere eines Alkylesters oder eines Glycerids der Fettsäure, handelt.

7. Verfahren nach Anspruch 6, **dadurch gekennzeichnet, dass** die Fettsäure aus Palmitoleinsäure, Ölsäure, Erucasäure und Nervonsäure ausgewählt wird und es sich bei der Fettsäure vorzugsweise um Ölsäure handelt.

8. Verfahren nach einem der Ansprüche 6 und 7, **dadurch gekennzeichnet, dass** sich die Fettsäure oder deren Glycerid von einem Pflanzenöl ableitet.

9. Verfahren nach Anspruch 6, **dadurch gekennzeichnet, dass** der Fettsäurealkylester durch Umesterung mindestens eines Pflanzenöls erhalten wird.

## Claims

1. A process for synthesizing hydroperoxy alcohols of formula (Ia) and/or (Ib):
R¹-CR⁴(OOH)-CR³(OH)-R² (Ia)
R²-CR³(OOH)-CR⁴(OH)-R¹ (Ib)
where:
R¹ and R² represent, each independently, an optionally substituted alkyl group containing from 1 to 20 carbon atoms or a group -L-A where L is a bond or a linear or branched alkylene chain, containing from 1 to 12 carbon atoms, optionally substituted, and A represents a hydrogen atom or a group -COXR in which X denotes an oxygen atom or a group -NR', and R and R' denote, each independently, a group selected from: a hydrogen atom, a C₁-C₆ alkyl group or a group -(CH₂)-CH(OCOR⁵)-CHOCOR⁶, R⁵ and R⁶ denoting, each independently, a linear or branched C₈-C₂₂ alkyl group optionally substituted and/or interrupted by at least one hydroperoxy and/or hydroxy group,
or else R¹ and R² together form a carbocycle consisting of 6 to 12 ring members and optionally substituted,
R³ and R⁴ represent, each independently, a hydrogen atom or an optionally substituted alkyl group containing from 1 to 20 carbon atoms or a group -L-A where L is a bond or a linear or branched alkylene chain, containing from 1 to 12 carbon atoms, optionally substituted, and A represents a hydrogen atom or a group -COXR in which X denotes an oxygen atom or a group -NR', and R and R' denote, each independently, a group selected from: a hydrogen atom, a C₁-C₆ alkyl group or a group -(CH₂)-CH(OCOR⁵)-CHOCOR⁶, R⁵ and R⁶ denoting, each independently, a linear or branched C₈-C₂₂ alkyl group optionally substituted and/or interrupted by at least one hydroperoxy and/or hydroxy group,
**characterized in that** it comprises a step of perhydrolysis of the epoxide of formula (II): 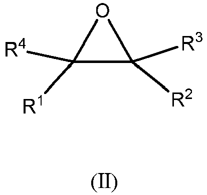
where:
R¹ and R² represent, each independently, an optionally substituted alkyl group containing from 1 to 20 carbon atoms or a group -L-A where L is a bond or a linear or branched alkylene chain, containing from 1 to 12 carbon atoms, optionally substituted, and A represents a hydrogen atom or a group -COXR in which X denotes an oxygen atom or a group -NR', and R and R' denote, each independently, a group selected from: a hydrogen atom, a C₁-C₆ alkyl group or a group -(CH₂)-CH(OCOR⁵)-CHOCOR⁶, R⁵ and R⁶ denoting, each independently, a linear or branched C₈-C₂₂ alkyl group optionally substituted and/or interrupted by at least one epoxy group,
or else R¹ and R² together form a carbocycle consisting of 6 to 12 ring members and optionally substituted,
R³ and R⁴ represent, each independently, a hydrogen atom or an optionally substituted alkyl group containing from 1 to 20 carbon atoms or a group -L-A where L is a bond or a linear or branched alkylene chain, containing from 1 to 12 carbon atoms, optionally substituted, and A represents a hydrogen atom or a group -COXR in which X denotes an oxygen atom or a group -NR', and R and R' denote, each independently, a group selected from: a hydrogen atom, a C₁-C₆ alkyl group or a group -(CH₂)-CH(OCOR⁵)-CHOCOR⁶, R⁵ and R⁶ denoting, each independently, a linear or branched C₈-C₂₂ alkyl group optionally substituted and/or interrupted by at least one epoxy group,
by reaction with an aqueous solution of hydrogen peroxide, in the presence of a catalyst consisting of phosphotungstic acid, said catalyst representing from 10 to 2000 ppm molar, relative to the number of moles of epoxide of formula (II), wherein the perhydrolysis reaction is carried out in the absence of organic solvent.

2. The process as claimed in claim 1, **characterized in that** the catalyst is used in an amount of at least 0.01 wt%, preferably at least 0.1 wt%, in particular at least 0.2 wt%, for example at least 0.4 wt% or at least 0.5 wt%, and for example at most 1.5 wt%, in particular at most 1 wt%, relative to the weight of epoxide.

3. The process as claimed in claim 1, **characterized in that** the catalyst is used in an amount of at least 50 ppm molar, for example at least 100 ppm molar, in particular at least 200 ppm molar, or even at least 400 ppm molar or even at least 800 ppm molar and at most 2000 ppm molar, advantageously at most 1500 ppm molar, or even at most 1000 ppm molar, relative to the molar amount of epoxide.

4. The process as claimed in any one of claims 1 to 3, **characterized in that** the hydrogen peroxide is used in an amount from 1 to 1.5 molar equivalent, preferably at a rate of 1.1 to 1.3 molar equivalent, relative to the epoxide.

5. The process as claimed in any one of claims 1 to 4, **characterized in that** the perhydrolysis reaction is carried out at a temperature from 5 to 60°C, preferably from 20 to 60°C, more preferably from 30 to 50°C, for a time from 10 minutes to 4 hours, for example from 45 minutes to 2.5 h.

6. The process as claimed in any one of claims 1 to 5, **characterized in that** the epoxide of formula (II) is the product of epoxidation of a mono- or polyunsaturated fatty acid or of an ester thereof, in particular an alkyl ester or a glyceride of said fatty acid.

7. The process as claimed in claim 6, **characterized in that** the fatty acid is selected from palmitoleic acid, oleic acid, erucic acid and nervonic acid, preferably the fatty acid is oleic acid.

8. The process as claimed in either one of claims 6 and 7, **characterized in that** the fatty acid or its glyceride is derived from a vegetable oil.

9. The process as claimed in claim 6, **characterized in that** the fatty acid alkyl ester is obtained by transesterification of at least one vegetable oil.
